(19) **Europäisches Patentamt / European Patent Office / Office européen des brevets**

(11) **EP 2 830 849 B1**

(12) **EUROPEAN PATENT SPECIFICATION**

(45) Date of publication and mention
of the grant of the patent:
**24.02.2016 Bulletin 2016/08**

(21) Application number: **13711405.4**

(22) Date of filing: **22.03.2013**

(51) Int Cl.:
**B29C 45/16** (2006.01)     **A61F 7/00** (2006.01)
**A61N 1/00** (2006.01)

(86) International application number:
**PCT/EP2013/056059**

(87) International publication number:
**WO 2013/144008 (03.10.2013 Gazette 2013/40)**

(54) **MOULDING METHOD**

FORMVERFAHREN

MÉTHODE DE MOULAGE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **27.03.2012 GB 201205387**

(43) Date of publication of application:
**04.02.2015 Bulletin 2015/06**

(73) Proprietor: **Mohn, Louise**
**0376 Oslo (NO)**

(72) Inventors:
• **BRIX, Ole**
**N-5892 Bergen (NO)**
• **ØVREEIDE, Espen**
**N-5302 Strusshamn (NO)**
• **NORDÅS, Ståle**
**N-5258 Blomsterdalen (NO)**
• **ELLINGSEN, Jan, Atle, Lossius**
**N-5251 Søreidgrend (NO)**

• **HENRIKSEN, Bård**
**N-5892 Bergen (NO)**
• **KLEPSVIK, Inge**
**N-5892 Bergen (NO)**

(74) Representative: **Enomoto, Kéi et al**
**RGC Jenkins & Co.**
**26 Caxton Street**
**London SW1H 0RJ (GB)**

(56) References cited:
| | |
|---|---|
| EP-A1- 1 357 774 | WO-A1-96/11098 |
| CN-Y- 201 371 557 | DE-A1- 10 212 794 |
| GB-A- 2 424 613 | JP-A- H0 241 221 |
| JP-A- S55 118 845 | JP-A- 2005 053 127 |
| US-A- 4 990 077 | US-A1- 2005 116 384 |
| US-A1- 2009 004 557 | US-A1- 2009 078 043 |
| US-A1- 2010 256 694 | US-A1- 2011 054 580 |
| US-A1- 2011 093 052 | US-A1- 2011 125 204 |
| US-A1- 2011 205 141 | |

**Description**

**FIELD OF THE INVENTION**

The present invention relates to a method of moulding a pad for applying stimulation to a human or animal body and the obtained pad.

**BACKGROUND OF THE INVENTION**

For a variety of therapeutic applications, several treatment modalities are currently known in the art including electrical stimulation, heat therapy and thermostimulation. Electrical stimulation involves the application of an electrical current to a single muscle or a group of muscles through one or more stimulation pads that are temporarily attached to the skin. A conductive gel is often used to improve the electrical conductivity between the stimulation pads and the skin. The muscle contraction that results from the applied electrical current can produce a variety of effects from strengthening injured muscles and reducing oedema to relieving pain and promoting healing. Heat therapy involves the application of heat to the body. Heat therapy is very useful as it has a number of effects such as relaxation of muscle spasm and increased blood flow that promotes healing. However, combination therapy, i.e. the synergistic use of other modalities such as massage, ultrasound and/or electrical stimulation has been found to be more effective than heat therapy alone. Thermostimulation is one such combination therapy that involves the use of heat therapy and electrical stimulation simultaneously. With thermostimulation, the healing benefits of heat are provided along with the strengthening, toning, pain relieving and healing benefits of electrical stimulation. Moreover, the application of heat has been found effective in that it allows the patient to tolerate higher currents. This yields higher electric field strengths, greater depths of penetration and, therefore, more positive results than could be achieved with electrical stimulation without heat. Thermostimulation can be performed using pads that are temporarily attached to the skin.

The inventors have identified a number of deficiencies with currently-available stimulation pads. There is a need for improved stimulation pads for electrical stimulation, heat therapy and thermostimulation. The inventors consider that an improved stimulation pad should: be able to deliver a sufficiently high electrical current to cause effective stimulation to the patient's muscles; be able to deliver sufficient heat to keep the patient's skin temperature constant at temperatures up to 43°C; have a high quality appearance; be possible to clean; be sufficiently flexible to allow good contact with the patient's skin; not cause irritation to the patient's skin; be watertight; and be durable.

Existing moulding methods are unable to produce stimulation pads that meet these exacting requirements. In particular, the inventors have found that, when existing moulding methods are used to mould one portion of an article that comprises a soft polymer against another portion of the article that also comprises a soft polymer, there is a tendency for flash to form at the interface of the two portions (which is detrimental to the appearance of the article), for the portions to deform (which is detrimental to the functionality and appearance of the article) or for the two portions not to bond to each other properly (which is detrimental to watertightness and durability). Furthermore, when electronic components are to be embedded within the article, there is a risk that the components could be damaged by the heat and pressure that are experienced during the moulding process.

WO 2011/150092 relates to a method of insert moulding of liquid silicone rubber. US7,739,791 relates to a method of producing an overmoulded electronic assembly. US6,319,448 relates to a process for the production of a measuring apparatus by total or partial overmoulding of its functional elements, particularly of its components and electric and electronic circuits.

US2005 116384 describes a method for manufacturing an in-old coating product. US2011 125204 describes an electrostimulation pad with an integrated temperature sensor. DE102005020200 describes the production of components for motor vehicles interior trim parts.

**SUMMARY OF THE INVENTION**

In accordance with an aspect of the present invention there is provided a method and a stimulation pad according to the accompanying claims.

A first aspect provides a method of moulding a stimulation pad comprising: forming a first portion of the article by injecting a first material into a first mould, the first mould being shaped so as to define a flange on the first portion of the article; placing the first portion of the article into a second mould, the second mould having a first plate and a second plate;

compressing the flange between the first and second plates of the second mould; and forming a second portion of the article by injecting a second material into the second mould whilst the flange is compressed between the first and second plates of the second mould.

The hardness of the first portion of the article increases when the flange is compressed. This increase in hardness makes the first portion of the article more able to withstand the compressive force that is experienced when the second portion of the article is formed, and thereby prevents deformation of the first portion.

Preferably, compressing the flange causes the flange to deform, thereby creating a seal between the flange and the second mould. The seal reduces the risk of flash on the parting line between the first and second plates of the second mould. This improves the visual appearance of the article and can avoid the need for a finishing process to remove flash.

Preferably, the first and second materials each comprise a soft plastic material. Preferably, the hardness of the first material and the hardness of the second material are each measurable on the Shore A durometer scale. At least one of the first material and the second material preferably has a Shore A hardness less than or equal to 85. When the first material is soft, the flange is able to deform when compressed between the first and second plates of the second mould. This creates a good seal between the flange and the second mould, and thereby reduces the risk of flash.

Preferably, both the first material and the second material have a Shore A hardness less than or equal to 85. When both the first and second materials are soft, the temporary increase in hardness that is achieved by compressing the flange allows one soft material to bond to another soft material without deformation of the first portion.

More preferably, at least one of the first material and the second material has a Shore A hardness of 45 to 70. More preferably, both the first material and the second material have a Shore A hardness of 45 to 70. In particular examples of the invention, both the first and second material have a Shore A hardness of 70.

Preferably, the first and second materials each comprise a polymer. More preferably, the first and second materials each comprise a thermoplastic material. More preferably, the first and second materials each comprise thermoplastic polyurethane. Thermoplastic polyurethane (TPU) is well suited for injection moulding, can be made relatively soft and exhibits good mechanical and chemical properties.

The method preferably further comprises placing an electrical circuit onto the first portion of the article prior to forming the second portion of the article. The electrical circuit is thereby encapsulated between the first and second portions of the article. The good bonding between the first and second portions of the article that results from the moulding method described herein prevents this ingress of water and other undesired substances into the circuit.

The method preferably further comprises covering at least a portion of the electrical circuit with a protective housing prior to forming the second portion of the article. The protective housing protects the circuit from the heat and pressure that are experienced when the second material is injected.

The second material is preferably injected into the second mould from an injection point that is oriented to direct the second material towards the protective housing. This results in the second material making contact with the protective housing before it makes contact with the circuit, and thereby reduces the risk of the circuit being damaged by the heat and pressure of the second material.

The circuit is preferably connected to a cable, wherein one end of the cable is covered by the protective housing and wherein the other end of the cable extends outside the protective housing. The protective housing protects the delicate connection between the circuit and the cable from the heat and pressure that are experienced when the second material is injected.

The cable preferably comprises a sheath, wherein the protective housing is adapted to grip the sheath. The protective housing thereby protects the connection between the circuit and the cable from being damaged by tensile forces that may be applied to the cable. The protective housing is preferably adapted to form a seal with the sheath. The seal between the protective housing and the sheath prevents the second material from entering the protective housing, and thereby prevents damage to the connection between the circuit and the cable.

Preferably, the circuit comprises a substrate and the protective housing is adapted to form a seal with the substrate. The seal between the protective housing and the substrate prevents the second material from entering the protective housing, and thereby prevents damage to the portion of the circuit that is contained within the protective housing.

Preferably the circuit is connected to a cable comprising a sheath and the second portion of the article is bonded to the sheath. The bonding of the second portion to the sheath prevents this ingress of water and other undesired substances into the circuit.

The article obtained by the method of the invention is a pad for applying stimulation to a human or animal body. The method described herein is particularly advantageous to manufacture a stimulation pad. For example, the use of soft materials results in a stimulation pad that is flexible and able to conform to the contours of the user's body, thereby allowing good contact to be made with the skin during stimulation. The stimulation pads have a high quality appearance due to the reduction of flash on the parting line between the first and second portions of the mould. Furthermore, the moulding method described herein results in a good bond between the first and second portions, which makes the stimulation pad durable, waterproof and easy to clean.

Preferably, the step of forming a first portion of the article comprises: placing an electrically conducting member into the first mould; and injecting the first material into the first mould, such that the first material bonds to the electrically conducting member. This results in a good bond between the electrically conducting member and the first material that forms the remainder of the first portion of the article.

The electrically conducting member preferably has an elliptical shape. An elliptically-shaped conducting member is less prone to being distorted by the compressive force that it experiences when the first material is injected. Reducing distortion of the conducting member is desirable in its own right and, additionally, helps to avoid flash forming at the interface of the conducting member and the first portion of the article.

The electrically conducting member preferably comprises a surface to which the first material is bonded, and a groove disposed substantially parallel to the surface. The electrically conducting member is usually harder than the first material. The groove allows the electrically conducting member to flex near to its interface with the first material, which reduces the risk of the first material detaching from the electrically conducting member when an applied force causes the stimulation pad to bend.

The first mould preferably comprises a ridge, and the groove is preferably adapted to engage with the ridge. The ridge thereby reinforces the conducting member when the first material is injected. This reduces distortion of the conducting member and thereby helps to avoid flash forming at the interface of the conducting member and the first portion of the article.

The electrical circuit preferably comprises a substrate having a slot extending therethrough, and the groove is preferably aligned with said slot when the electrical circuit is placed onto the first portion of the article. When the second material is injected, it can pass through the slot and bond with the interior surface of the groove. This improves the durability of the stimulation pad, by allowing the second portion to bond to the conducting member and by holding the circuit in position.

The first mould preferably comprises an insert, the insert being separated from the first mould by a gap, wherein the gap allows air to exit the first mould when the first material is injected into the first mould. By allowing air to exit the first mould in this manner, the first material is able to fill the whole of the first mould.

A further aspect of the invention provides a stimulation pad produced by the method described herein.

## BRIEF DESCRIPTION OF THE DRAWINGS

Preferred features of the invention will now be described, purely by way of example, with reference to the accompanying drawings, wherein like elements are indicated using like reference signs, and in which:

Figure 1 is a top isometric view of a stimulation pad;

Figure 2 is a bottom isometric view of the stimulation pad shown in Figure 1;

Figure 3 is an exploded view of the stimulation pad shown in Figures 1 and 2;

Figure 4 is the top isometric view of the stimulation pad shown in Figures 1 to 3, in which a flange is highlighted;

Figure 5 is a flow diagram of a method of moulding the stimulation pad shown in Figures 1 to 4;

Figure 6 is a cross-sectional view of a first mould during the first step of the method shown in Figure 5;

Figure 7 is an isometric view of a first plate of the first mould shown in Figure 6;

Figure 8 is an isometric view of a second plate of the first mould shown in Figure 6;

Figure 9 is a cross-sectional view of the first mould when it has been closed;

Figure 10 is a cross-sectional view of the first mould during the second step of the method shown in Figure 5;

Figure 11 is a magnified view of Figure 10;

Figure 12 is a plan view of the stimulation pad during the second step of the method shown in Figure 5;

Figure 13 is a top isometric view of a first portion of the stimulation pad that results from the second step of the method shown in Figure 5;

Figure 14 is a top isometric view of the first portion of the stimulation pad shown in Figure 13 following the addition of electronic components;

Figure 15 is a cross-sectional view of a second mould during the third step of the method shown in Figure 5;

Figure 16 is an isometric view of a first plate of the second mould shown in Figure 15;

Figure 17 is an isometric view of a second plate of the second mould shown in Figure 15;

Figure 18 is a cross-sectional view of the second mould during the fourth step of the method shown in Figure 5;

Figure 19 is a magnified view of Figure 18;

Figure 20 is a cross-sectional view of the second mould during the fifth step of the method shown in Figure 5;

Figure 21 is a magnified view of Figure 20;

Figure 22 is an isometric view of a conducting member of the stimulation pad shown in Figures 1 to 3;

Figure 23 is a partial cross-sectional perspective view of a conducting member of the stimulation pad shown in Figures 1 to 3;

Figure 24 is a cross-sectional view of the stimulation pad shown in Figures 1 to 4;

Figure 25 is a schematic diagram of a stimulation system comprising the stimulation pad shown in Figures 1 to 4;

Figure 26 is a top plan view of the circuit shown in Figure 3;

Figure 27 is a bottom plan view of the circuit shown in Figure 3; and

Figure 28 is a schematic diagram of a connector for the circuit shown in Figure 3.

## DETAILED DESCRIPTION

The present invention relates to a method of moulding a pad for applying stimulation to a human or animal body. The Invention further refers to stimulation pads obtained by the method of the invention.

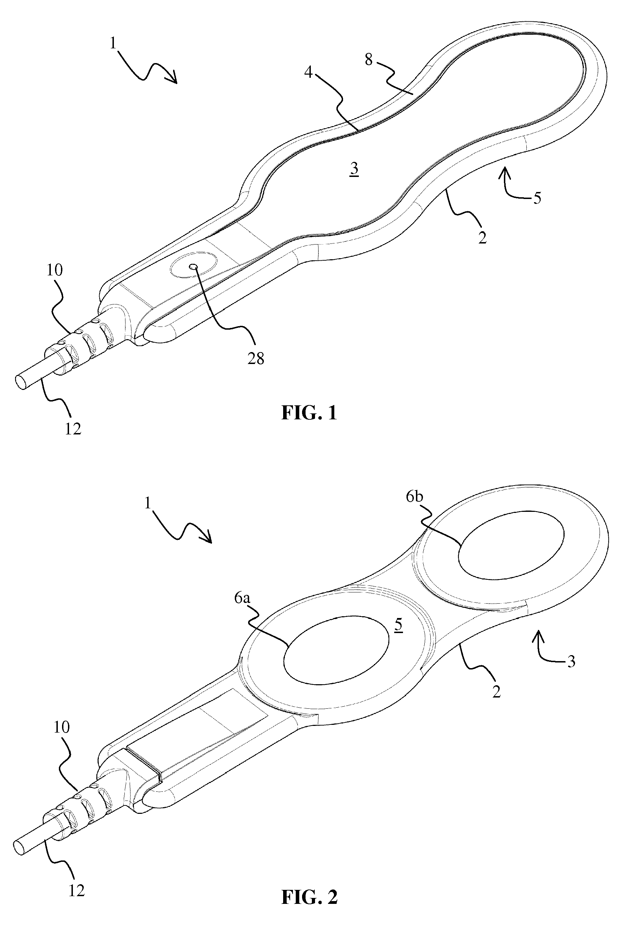

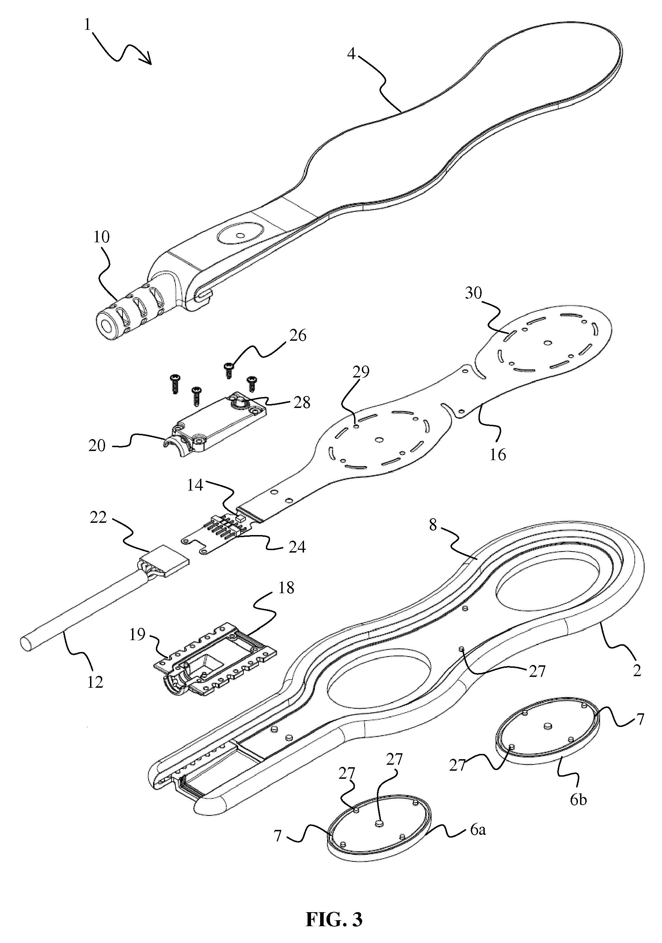

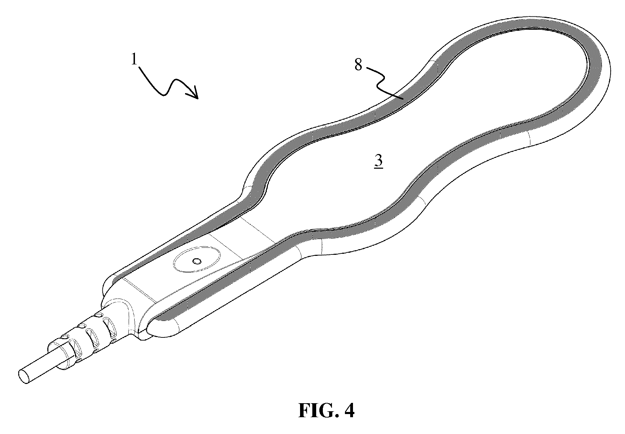

A stimulation pad 1 will now be described with reference to Figures 1 to 4. Figures 1 and 2 are top and bottom isometric views respectively of the stimulation pad 1. Figure 3 is an exploded view of the stimulation pad 1. The stimulation pad 1 comprises a first portion 2 and a second portion 4. The first portion 2 is bonded to the second portion 4 by the moulding method that is described below. The stimulation pad 1 has a first surface 3, shown facing upwards in Figure 1, which is defined by both the first portion 2 and the second portion 4. The stimulation pad 1 also has a second surface 5, shown facing upwards in Figure 2, which is primarily defined by the first portion 2. The first surface 3 is oriented in substantially the opposite direction to the second surface 5.

The first portion 2 of the stimulation pad 1 comprises a flange 8. The flange 8 is shown by the shaded area in Figure 3. The flange 8 forms part of the first surface 3 of the stimulation pad 1. The flange 8 surrounds at least part of the perimeter of the second portion 4 of the stimulation pad 1.

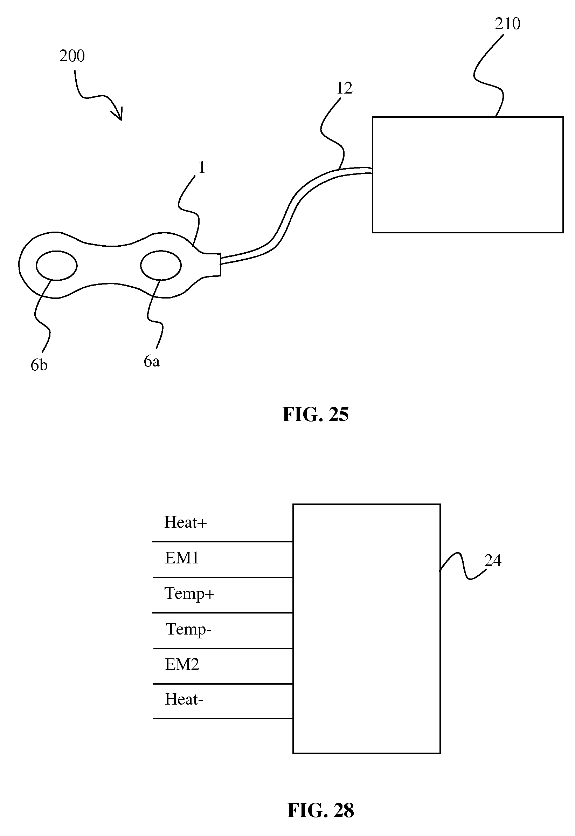

Various components 12, 14, 16, 18, 20, 22, 24, 26 are located inside the stimulation pad 1, and are encapsulated between the first portion 2 and the second portion 4. These components include an electrical circuit 16 that is operable to generate stimulation that can be provided to a human or animal body. For example, the circuit 16 can generate electrical stimulation and/or heat. The circuit 16 may comprise a first connector 24 for connection to a corresponding second connector 22 of a cable 12. The cable 12 extends outside the stimulation pad 1, such that only part of its length is contained within the stimulation pad 1. The cable 12 comprises a core surrounded by a sheath, the core comprising one or more electrically conducting wires. The cable 12 is operable to provide electrical power and, optionally, one or more control signals, from a console (indicated by reference numeral 210 in Figure 25) to the circuit 16. The cable 12 may also be operable to provide one or more feedback signals from the circuit 16 to the console.

A strain relief 10 preferably surrounds the cable 12 at its point of entry into the stimulation pad 1. The strain relief 10 restricts the bending of the cable 12, and thereby reduces the risk of bending causing damage to the cable 12 itself or to the first and second connectors 22, 24. The strain relief 10 is preferably integrally formed with the second portion 4 of the stimulation pad 1. The strain relief 10 is preferably bonded to the cable 12 by the moulding method that is described below.

The circuit 16 may also comprise a visual indicator 14. The visual indicator may comprise a light emitting diode (LED), for example. The visual indicator 14 is operable to provide a visible indication of the status of the circuit 16. For example, the visual indicator 14 may indicate that electrical power is being supplied to the circuit 16 and/or may indicate that the circuit 16 is ready to apply stimulation to a human or animal body. As the visual indicator 14 is located inside the stimulation pad 16, the second protective housing 20 may comprise a transparent region 28 that penetrates the second portion 4, thereby allowing a visual indication provided by the visual indicator 14 to be seen from outside the stimulation pad 16. The whole of the second protective housing 20 is preferably formed from a transparent material, such that the transparent region 28 is defined by a projection formed on the surface of the second protective housing 20.

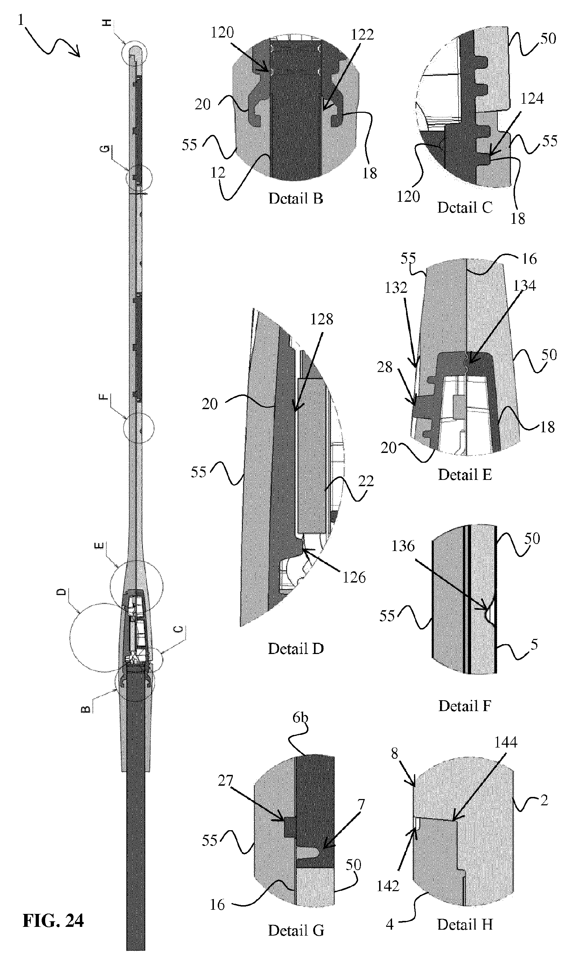

The first connector 24, second connector 22 and visual indicator 14 are preferably disposed between a first protective housing 18 and a second protective housing 20. The first and second protective housings 18, 20 protect the connectors 22, 24 and visual indicator 14 from the heat and pressure that are experienced during the formation of the second portion 4 by the moulding method that is described below. Additionally, the first and second protective housings 18, 20 protect the connectors 22, 24 against damage from tensile forces that may be applied via the cable 12 (as discussed in more detail below, with reference to Detail B of Figure 24). One or more screws 26 can be provided to fasten the first protective housing 18 to the second protective housing 20.

The stimulation pad 1 comprises two electrically conducting members 6a, 6b. The conducting members 6a, 6b are provided on the second surface 5. The first portion 2 of the stimulation pad 1 surrounds each conducting member 6a, 6b. The first portion 2 is bonded to the conducting members 6a, 6b by the moulding method that is described below. The purpose of the conducting members 6a, 6b is to effect electrical stimulation by conducting electricity from the circuit 16 to a human or animal body placed in contact with the conducting members 6a, 6b. The stimulation pad 1 could comprise just one conducting member, more than two conducting members or, in the case of a stimulation pad that does not provide electrical stimulation, no conducting members at all. Each conducting member 6a, 6b comprises a groove 7. Each conducting member 6a, 6b preferably further comprises one or more pins 27.

The circuit 16 is formed on a substrate that comprises one or more holes 29 and/or one or more slots 30 that extend through the substrate. The holes 29 align with the pins 27 on the conducting members 6a, 6b when the stimulation pad 1 is assembled. The slots 30 align with the grooves 7 of the conducting members 6a, 6b when the stimulation pad 1 is assembled. Whilst the slots 30 are preferably elongated in the plane of the substrate, as shown in Figure 3, the slots 30 could alternatively have a circular cross-section (i.e. the slots 30 could be round holes).

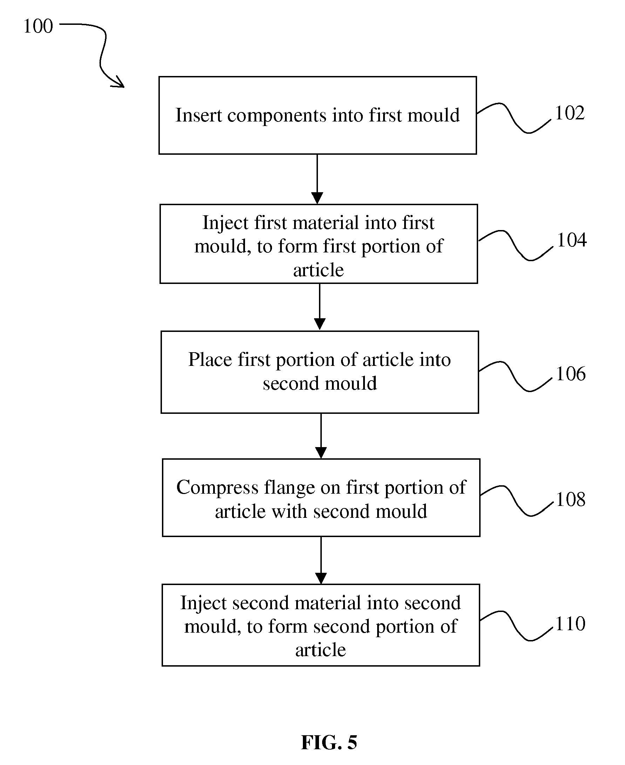

A method 100 of manufacturing the stimulation pad 1 will now be described with reference to Figures 5 to 21.

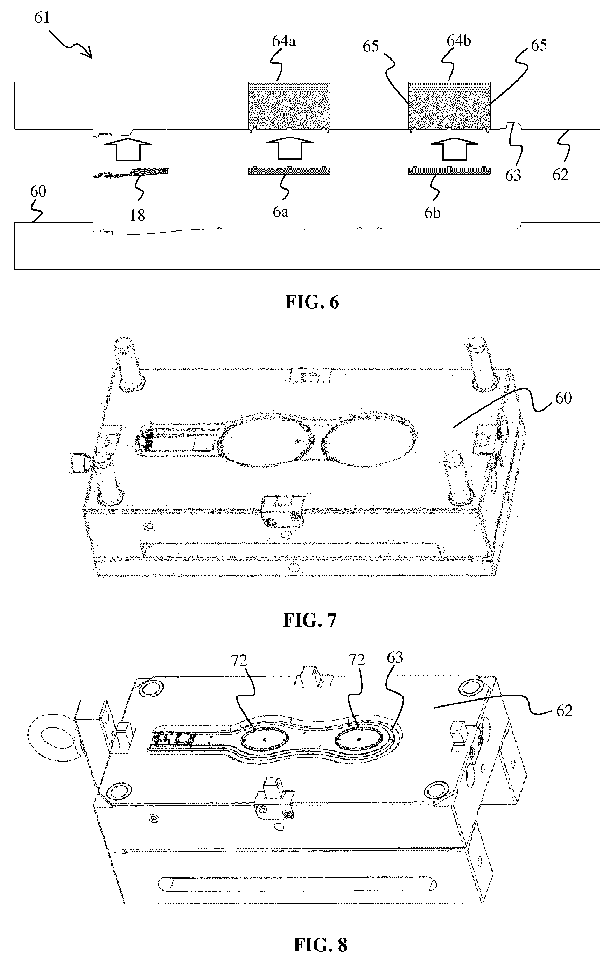

The method 100 starts at step 102, in which components 6, 18 are inserted into a first mould 61. Step 102 is illustrated by Figure 6. The first mould 61 comprises a first plate 60 and a second plate 62. Isometric views of the first plate 60 and the second plate 62 are shown in Figures 7 and 8 respectively. The surface of the second plate 62 corresponds to the shape of the components that are to be inserted into the first mould 61. Thus, the conducting members 6a, 6b and the first protective housing 18 are inserted into the first mould 61 by positioning them on the surface of the second plate 62, as illustrated by the arrows in Figure 6. Step 102 is optional, and need not be performed if the article to be moulded does not require any inserts. The conducting members 6a, 6b and the first protective housing 18 are formed prior to step 102, preferably by a moulding process.

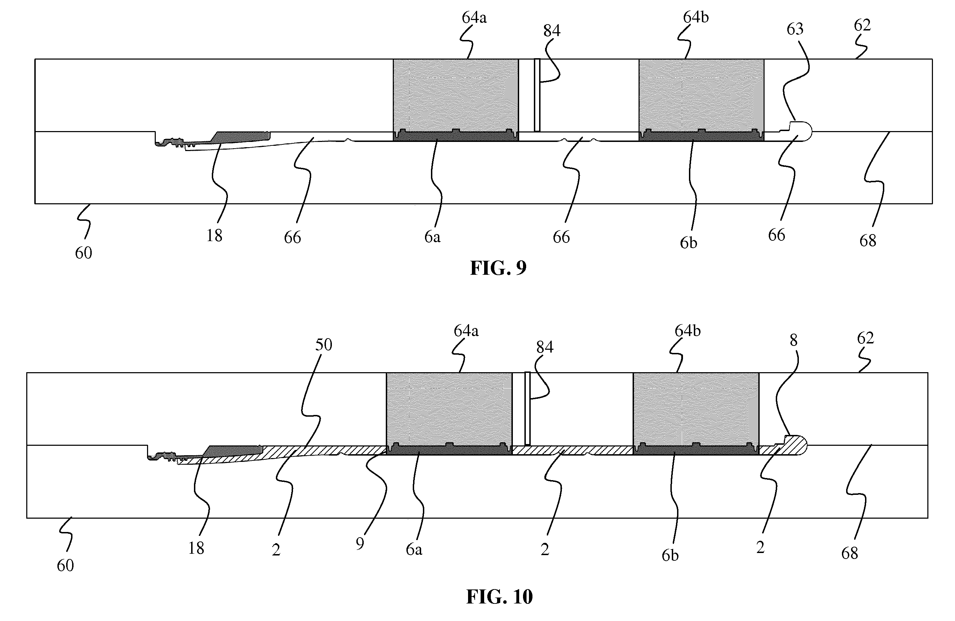

The first mould 61 is then closed, as illustrated by Figure 9. When the first mould 61 is closed, a cavity 66 is defined by the first plate 60, the second plate 62, the conducting members 6a, 6b and the first protective housing 18. A parting line 68 is defined by the abutting surfaces of the first plate 60 and the second plate 62. The first mould 61 is shaped so as to define the flange 8 on the first portion 2. More specifically, the second plate 62 comprises a recess 63 that defines the flange 8 when material is injected into the cavity 66.

In step 104, a first material 50 is injected into the cavity 66 of the first mould 61, thereby forming the first portion 2. Step 104 is illustrated by Figures 10 to 12. The hatched area in Figure 10 shows the first portion 2 of the stimulation pad 1. Figure 10 illustrates that a flange 8, having a shape corresponding to the shape of the recess 63, is formed when the first material 50 is injected into the cavity 66 of the first mould 61.

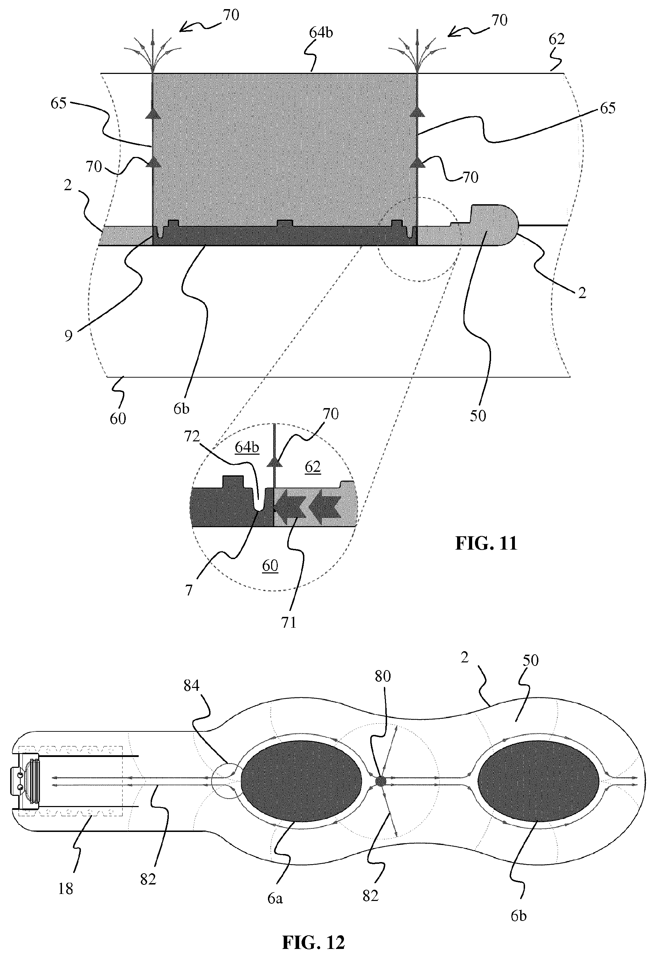

Figure 11 is a magnified view of Figure 10, to illustrate how air leaves the first mould 61 when the first material 50 is injected into the cavity 66 in step 104. The second plate 62 comprises one or more inserts 64a, 64b. A gap 65 exists between the inserts 64 and the surrounding regions of the second plate 62. Air can exit the cavity 66 through the gap 65 when the first material 50 is injected into the cavity 66. The escape of air from the cavity 66 is illustrated by the arrows 70. However, the gap 65 is too narrow to allow the first material 50 to exit the cavity 66. For example, the gap 65 can have a thickness of 0.005 millimetres, although other suitable thicknesses are possible. Allowing air to escape in this manner avoids air being trapped in the cavity 66, which allows the first material 50 to fill the whole of the cavity 66 and improves bonding between the first material 50 and the conducting members 6a, 6b. Allowing air to escape also avoids the Diesel effect, whereby compressed air that is trapped in the cavity 66 could cause the first material 50 to burn.

Each insert 64a, 64b comprises a ridge 72. The shape of each ridge 72 corresponds to the shape of the groove 7 in a respective conducting member 6a, 6b, such that each ridge 72 engages with a respective groove 7 when the conducting members 6a, 6b are inserted into the first mould 61 in step 102. As can be seen in Figure 8, each ridge 72 has an elliptical shape, thereby allowing engagement with the entirety of a groove 7, which also has an elliptical shape. The ridges 72 reinforce the conducting members 6a, 6b, thereby preventing the conducting members 6a, 6b from being deformed by the force that is exerted upon them when the first material 50 is injected into the cavity 66 in step 104. The direction of the force exerted upon the conducting members 6a, 6b by the first material during step 104 is illustrated by the arrow 71 in Figure 11. Alternatively or in addition, the conducting members 6a, 6b can be compressed between the first plate 60 and the second plate 62 of the first mould 61, so as to increase the hardness of the conducting members 6a, 6b and thereby prevent them from being deformed by the force experienced when the first material 50 is injected into the cavity 66 in step 104. These measures to prevent deformation of the conducting members 6a, 6b also help to prevent flash (i.e. unwanted excess material) forming around the conducting members 6a, 6b when the first material 50 is injected.

Figure 12 shows the flow path 82 of the first material 50 during step 104. The first material 50 is in the molten state when it is injected into the first mould 61. The first material 50 is injected into the first mould 61 at an injection point 80. A vertical injection machine is preferably used to inject the first material 50. The injection point 80 is preferably located near to the centre of the cavity 66, so as to reduce the distance over which the first material 50 has to flow, thus lowering the pressure needed to fill the first mould 61 completely. The flow path 82 in the region denoted by reference numeral 84 shows that the elliptical shape of the conducting members 6a, 6b helps to reduce the approach angle of the first material 50, thus resulting in a more laminar flow of the first material 50 around the conducting members 6a, 6b. This helps to minimise deformation of the conducting members 6a, 6b when the first material 50 is injected.

When injected into the first mould 61, the first material 50 bonds to the conducting members 6a, 6b and the first protective housing 18. This results in a watertight seal between the conducting members 6a, 6b and the surrounding regions of the first portion 2. This advantageously prevents sweat and conductive gel from entering the stimulation pad 1 during use, which may cause the circuit 16 to malfunction. Each side of the first protective housing 18 preferably comprises a plurality of holes 19 (shown in Figure 3). The holes 19 increase the bonding strength of the first protective housing 18 to the first material 50, and thereby reduce the risk of the first protective housing 18 being displaced by the force experienced during injection of a second material during step 110.

The optimum pressure, flow rate and temperature at which to inject the first material 50 during step 104 will depend on numerous factors, such as the properties of the first material 50, the geometry of the cavity 66 and the ability of air to exit the cavity 66. In general, higher pressure, flow rate and temperature improves the bonding between the first material 50 and the conducting members 6a, 6b, but increases the risk of flash forming around the conducting members 6a, 6b. Thus, the optimum pressure, flow rate and temperature are found through trial and terror, so as to find the maximum values for these parameters that do not result in flash.

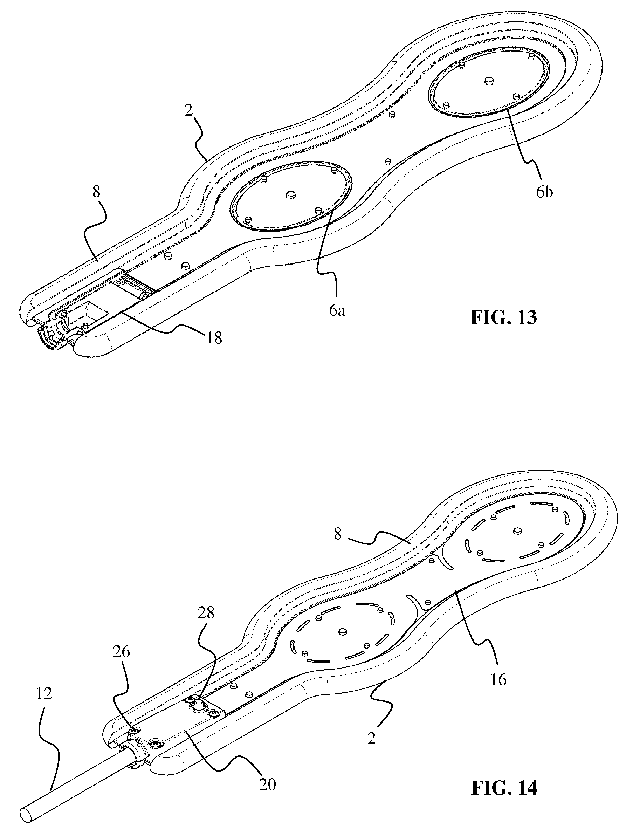

When the first material 50 has cooled, the first portion 2 is removed from the first mould 61. Figure 13 shows the first portion 2 immediately after step 104. The first material 50 is bonded to the conducting members 6a, 6b and the first protective housing 18 by the moulding process. A circuit assembly (comprising circuit 16, first connector 24, second connector 22 and cable 12) is then positioned on the first portion 2. The second protective housing 24 is then secured to the first protective housing 18 by screws 26, which results in the assembly shown in Figure 14. The method then proceeds to step 106.

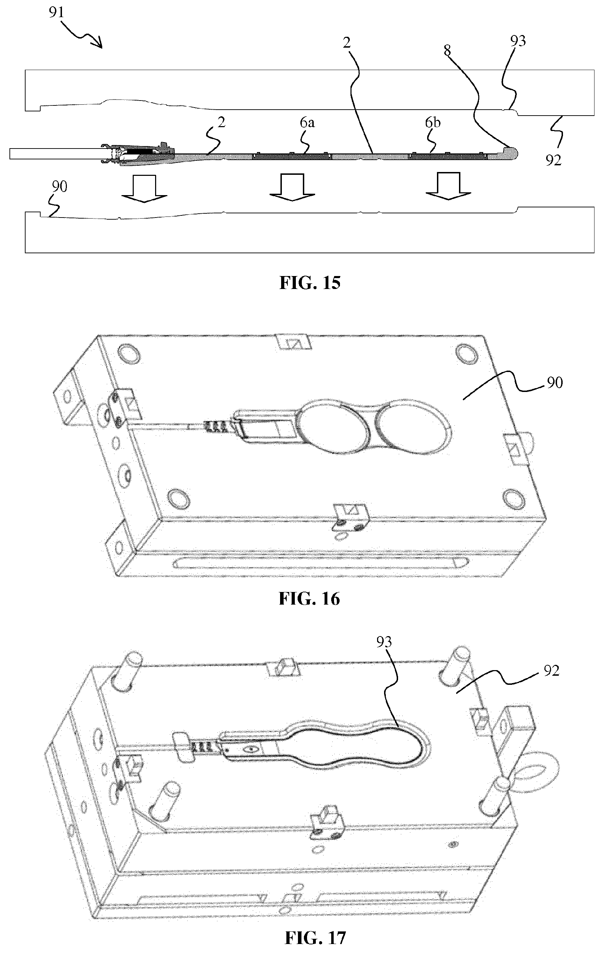

In step 106, the first portion 2 is placed into a second mould 91. Step 106 is illustrated by Figure 15. The second mould 91 comprises a first plate 90 and a second plate 92. Isometric views of the first plate 90 and the second plate 92 are shown in Figures 16 and 17 respectively. The surface of the first plate 90 corresponds to the shape of the second surface 5 of the stimulation pad 1. Thus, the first portion 2 (which constitutes the majority of the second surface 5) is placed into the second mould 91 by positioning it on the surface of the first plate 90, as illustrated by the arrows in Figure 15.

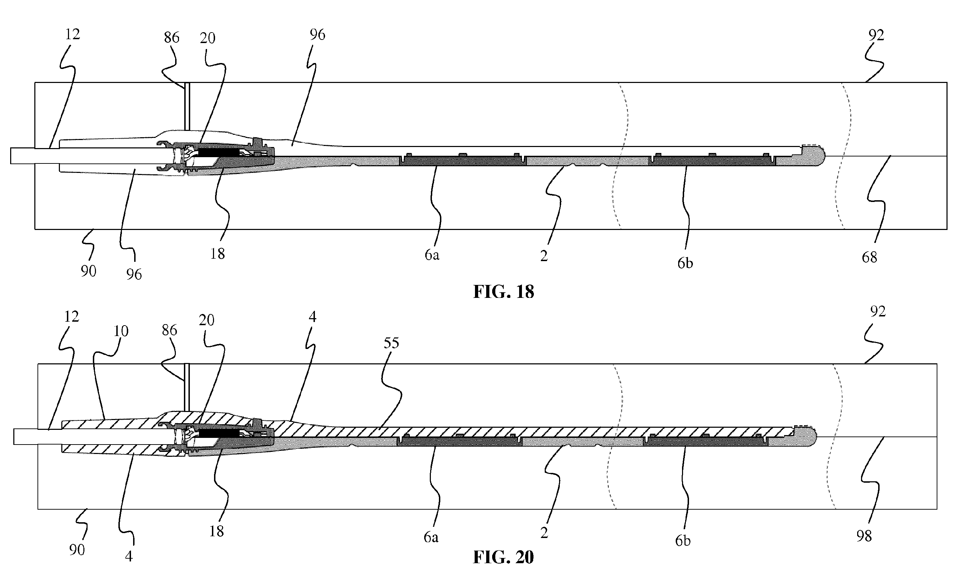

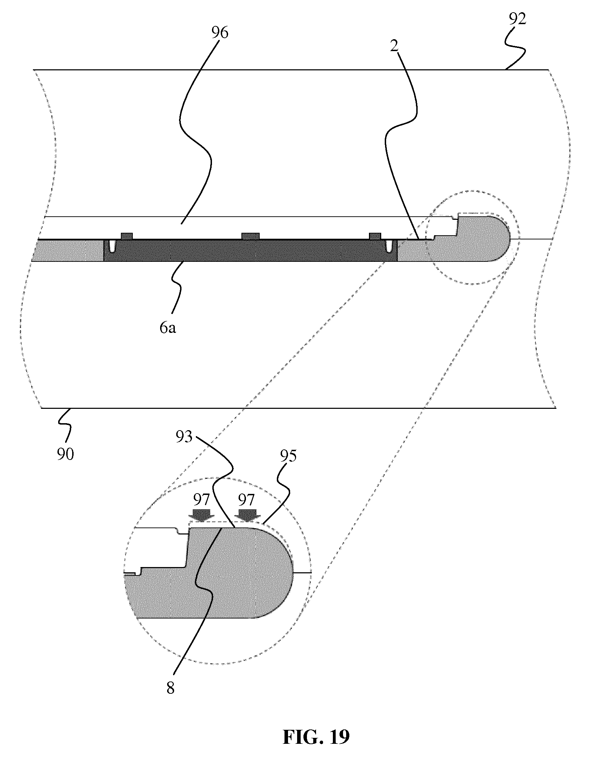

In step 108, the flange 8 is compressed between the first plate 90 and the second plate 92 of the second mould 91. Step 108 is illustrated by Figures 18 and 19. The flange 8 is compressed by closing the second mould 91. The distance between the first plate 90 and the second plate 92, measured at the point 93 at which the second plate 92 abuts the flange 8 when the second mould 91 is closed, is less than the thickness of the first portion 2 at the flange 8. Thus, closing the second mould 91 causes the flange to be compressed between the first plate 90 and the second plate 92. This can be seen clearly in Figure 19, which is a magnified view of Figure 18. The dashed line 95 illustrates the profile of the flange 8 in the absence of a compressive force. The compressive force 97 that is applied to the flange 8 when the second mould 91 is closed causes the flange 8 to deform. Compression of the flange 8 creates a seal between the first portion 2 and the second plate 92. Compression of the flange 8 also causes the hardness of the first portion 2 to increase, as described in more detail below. The surface of the flange 8 is substantially parallel to the surface of the second plate 92 at all points 93 at which the which the second plate 92 abuts the flange 8 when the second mould 91 is closed. The compressive force 97 is substantially perpendicular to the surface of the flange 8.

When the second mould 91 is closed, a cavity 96 is defined by the first plate 90, the second plate 92 and the first portion 2. A parting line 98 is defined by the abutting surfaces of the first plate 90 and the second plate 92.

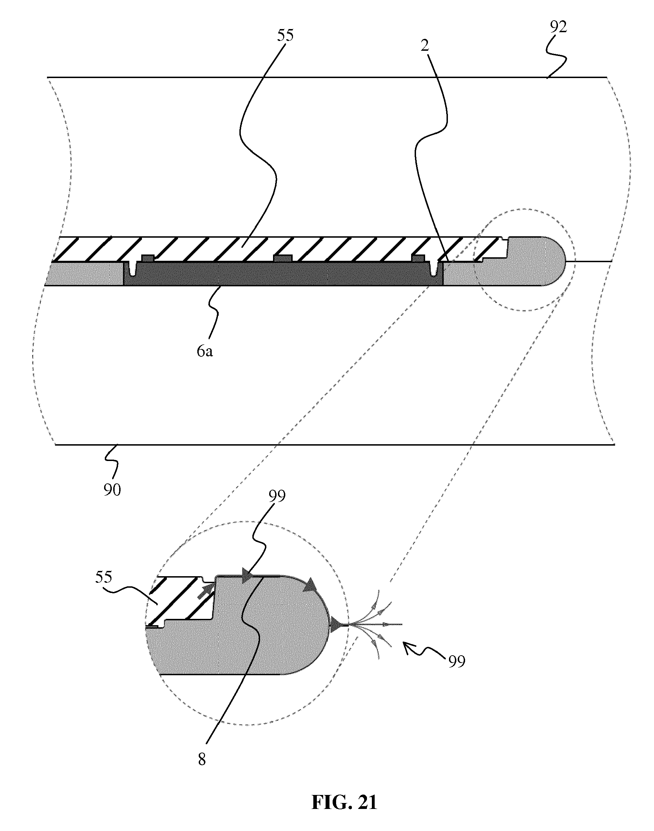

In step 110, a second material 55 is injected into the cavity 96 of the second mould 91 whilst the flange 8 is compressed between the first plate 90 and the second plate 92, thereby forming the second portion 4. Step 110 is illustrated by Figures 20 and 21. The hatched area in Figures 20 and 21 denotes the second portion 4. Figure 21 is a magnified view of Figure 20, to illustrate how air leaves the second mould 91 when the second material 55 is injected into the cavity 96 in step 110. As previously mentioned, compression of the flange 8 creates a seal between the first portion 2 and the second plate 92. This seal prevents the second material 55 from exiting the cavity 96, and thereby reduces the risk of flash forming on the parting line 98. However, the seal allows air to exit the cavity 96, by flowing between the flange 8 and the second plate 92, as illustrated by arrows 99. This avoids air being trapped in the cavity 96, which allows the second material 55 to fill the whole of the cavity 96 and improves bonding between the first material 50 and the second material 55. Allowing air to escape also avoids the Diesel effect, whereby compressed air that is trapped in the cavity 96 could cause the second material 55 to burn.

The second material 55 is injected into the second mould 91 at an injection point 86. The second material 55 is in the molten state when it is injected into the cavity 96. A vertical injection machine is preferably used to inject the second material 55. The injection point 86 is located as far away from the circuit 16 as possible, so as to reduce the risk of the electrical components of the circuit 16 being damaged by the heat and pressure of the second material 55, which are greatest near to the injection point. Thus, the injection point 86 is preferably located above the second protective housing 20. Furthermore, the injection point 86 is preferably oriented so as to direct the second material 55 towards the second protective housing 20 when it first enters the cavity 96. The first and second protective housings 18, 20 protect the connectors 22, 24 and visual indicator 24 against damage from the heat and pressure that are experienced when the second material 55 is injected. Furthermore, by locating the injection point 86 above the second protective housing 20, the pressure applied by the incoming second material 55 when it makes contact with the circuit 16 forces the circuit 16 towards the first portion 2. This reduces the risk of the second material 55 flowing underneath the circuit 16, which would have the undesirable effect of preventing the circuit 16 making good electrical contact with the conducting members 6a, 6b. The injection point 86 is most preferably located above the region of the second protective housing 20 that surrounds the cable 12. The presence of the cable 12 stiffens this region of the second protective housing 20, and thereby improves the ability of the second protective housing 20 to withstand the pressure applied by the incoming second material 55. Furthermore, locating the injection point 86 closer to the cable 12 helps to improve the bonding between the second material 55 and the sheath of the cable 12, by reducing the flow distance and thus ensuring that the second material 55 is relatively warm when it makes contact with the sheath.

When injected into the second mould 91, the second material 55 bonds to the first material 50. This results in a watertight seal between the first portion 2 and the second portion 4. This advantageously prevents sweat and conductive gel from entering the stimulation pad 1 during use, which may cause the circuit 16 to malfunction. The second material 55 also bonds to the second protective housing 20. Since one component (i.e. the second portion 4) is moulded upon another component (i.e. the first portion 2), step 110 may be refereed to as an overmoulding process. Similarly to step 104, the optimum pressure, flow rate and temperature at which to inject the second material 55 during step 110 are found through trial and terror, so as to find the maximum values for these parameters that do not result in flash.

As mentioned previously, the grooves 7 of the conducting members 6a, 6b are aligned with the slots 30 in the substrate of the circuit 16. When the second material 55 is injected in step 110, the second material 55 passes through the slots 30 and bonds with the interior surface of the grooves 7. This improves the durability of the stimulation pad 1, by allowing the second portion 4 to bond to the conducting members 6a, 6b and by holding the circuit 16 in position. The grooves 7 increase the available surface area of the conducting members 6a, 6b to which the second material 55 can bond.

The strain relief 10 is defined by the shape of the second mould 91. Thus, the strain relief 10 is formed by injection of the second material 55 into the second mould 91 in step 110. The second material 55 bonds to the sheath of the cable 12 in the region of the strain relief 10. This forms a watertight seal between the cable 12 and the strain relief 10.

When the second material 55 has cooled, the stimulation pad 1 is removed from the second mould 91. This results in the stimulation pad 1 shown in Figures 1 to 3.

Figure 24 illustrates preferred features of the stimulation pad 1, any or all of which can be provided. As shown in Detail B, the inner surfaces of the first protective housing 18 and the second protective housing 20 each comprise one or more ribs 120 to engage with the sheath of the cable 12. The ribs 120 grip the sheath and hold the cable 12 in position. Thus, tensile forces applied to the cable 12 are borne by the sheath and the ribs 120, rather than by the connectors 22, 24. This protects the connectors 22, 24 from being damaged by tensile forces applied to the cable 12, and reduces the risk of the cable 12 being detached from the stimulation pad 1. The ribs 120 also form a seal with the cable 12, which prevents the second material 55 from entering the first protective housing 18 and the second protective housing 20 during step 110, and thereby prevents damage to the connectors 22, 24. The first protective housing 18 and the second protective housing 20 are shaped so as to anchor them to the second material 55. For example, as shown in Detail B, the first protective housing 18 and the second protective housing 20 may comprise a hook-shaped portion 122. As another example, shown in Detail C, the outer surface of the first protective housing 18 comprises one or more ridges 124 for engagement with the first material 50 and the second material 55. Similar ridges could be provided on the second protective housing 20.

As shown in Detail D of Figure 24, the second protective housing 20 comprises a lip 126 for engagement with the second connector 22. The lip 126 prevents the second connector 22 being detached from the first connector 24 when a force is applied to the cable 12.

As shown in Detail E of Figure 24, the first protective housing 18 and the second protective housing 20 comprise one or more elongated corrugations 134. The corrugations 134 form a seal on each side of the substrate of the circuit 16, so as to prevent the second material 55 from entering the first protective housing 18 and the second protective housing 20 during step 110. The circuit 16 preferably has a flexible substrate, which is caused to deform by the corrugations 134, thereby improving the quality of the seal. Detail E also shows that the transparent region 28 is defined by a projection formed on the surface of the second protective housing 20. In addition to allowing the visual indicator 14 to be seen from outside the stimulation pad 1, this projection preferably has the additional purpose of holding the second protective housing 20 in position when the second material 55 is injected into the second mould 91 in step 110. To achieve this additional purpose, the projection is shaped so as to abut the internal surface of the second plate 92 when the second mould 91 is closed.

As shown in Detail F of Figure 24, the second surface 5 of the stimulation pad 1 comprises one or more grooves 136 to increase the flexibility of the pad 1, thereby improving the ability of the pad to conform to the contours of the body. The grooves 136 also reduce the tendency for sweat and conductive gel to form a short circuit between the conducting members 6a, 6b, which would reduce the efficacy of electrical stimulation.

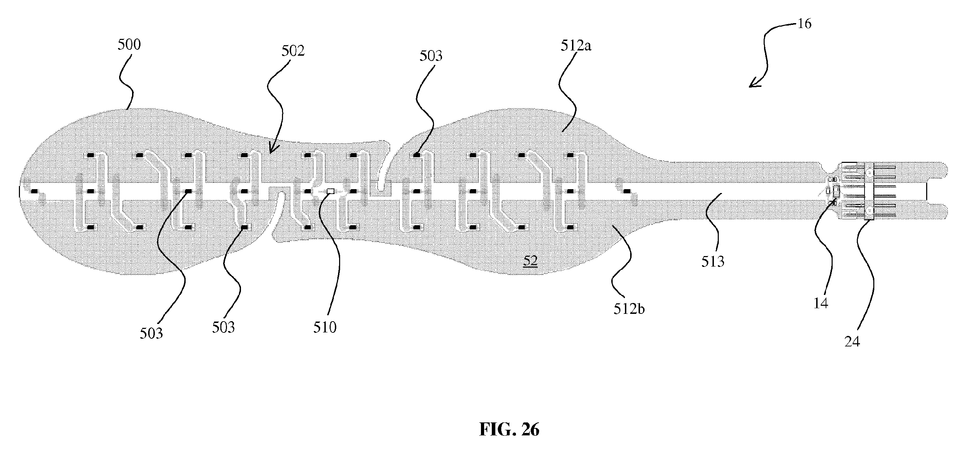

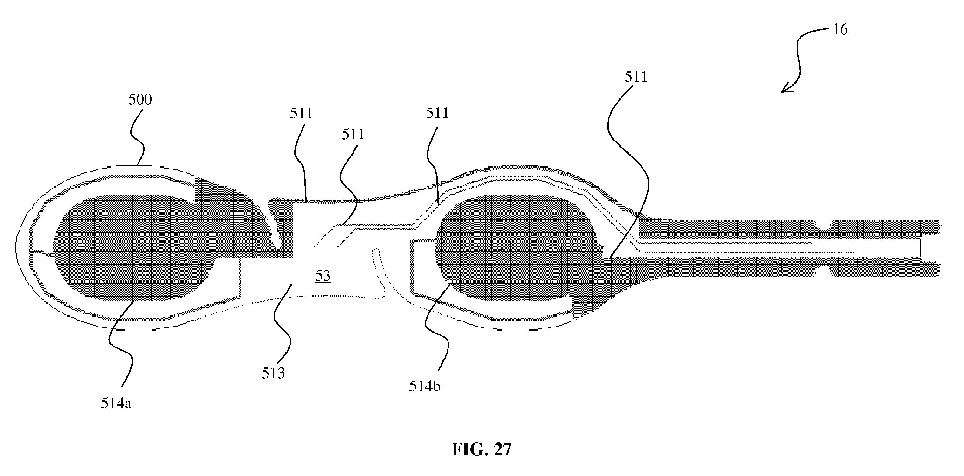

As shown in Detail G of Figure 24, the first portion 2 and the conducting members 6a, 6b comprise one or more pins 27. The pins 27 engage with corresponding holes 29 (shown in Figure 3) in the circuit 16. The engagement of the pins 27 with the holes 29 helps to keep the circuit 16 in position during step 110, when the circuit 16 experiences large forces as the second material 55 is injected. Furthermore, the pins 29 bond with the second material 55. The bonding of the pins 29 to the second material 55 is particularly advantageous because the substrate of the circuit 16 (described below, with reference to Figures 26 and 27) is usually formed from a material that will not bond to either the first material 50 or the second material 55. Thus, the pins 29 provide additional bonding points between the second portion 4 and the first portion 2 or the conducting members 6a, 6b, thereby improving the durability of the stimulation pad 1. Detail G also shows the groove 7 in the conductive member 6b.

As shown in Detail H of Figure 24, the surface of the second portion 4 comprises a depression 142 adjacent its interface with the flange 8. The depression 142 reduces the risk of flash forming during step 110. However, if any flash is formed during step 110, the depression 142 reduces the visibility of the flash. The second mould 91 is shaped so as to define the depression 142 on the surface of the second portion 4.

The first material 50 and the second material 55 are both electrically insulating materials. This ensures that the conducting members 6a, 6b are the only regions on the external surface of the stimulation pad 1 that are able to conduct electricity. The first material 50 may be the same as the second material 55. Alternatively, the first material 50 may be different from the second material 55.

The first material 50 and the second material 55 each comprise a soft polymer. In this context, the term "soft" is preferably understood to mean that the hardness of the polymer is measurable on the Shore A durometer scale. The term "soft" is more preferably understood to mean that the hardness of the polymer is below 85 Shore A. Due to the softness of the first material 50, there is a risk that the first material 50 could be deformed by the compressive force that it experiences when the second material 55 is injected. Deformation of the first material 50 would be detrimental to the appearance and functionality of the article produced by the moulding method, and could also increase the risk of flash forming when the second material 55 is injected in step 100. The risk of the first material 50 deforming is mitigated by compressing the flange 8 during steps 108 and 110. Compression of the flange 8 causes a reversible, temporary increase in the hardness of the first portion 2, particularly its hardness in the vicinity of the flange 8. The first portion 2 returns to its original hardness (i.e. becomes softer) when the compression is released. This increase in hardness allows the first material 50 to withstand the compressive force that is experienced when the second material 55 is injected, which results in a high quality article being produced by the moulding method.

The compression of the flange 8 during steps 108 and 110 is particularly advantageous when the first material 50 is different from the second material 55. The first material 50 and the second material 55 may comprise the same soft polymer, but each material may comprise different additives. For example, the first and second materials 50, 55 may comprise different fillers or plasticisers, so as to give the first portion 2 different properties from the second portion 4. As another example, the first and second materials 50, 55 may comprise different colorants, so as to give the first portion 2 a different colour from the second portion 4. Alternatively, the first material 50 and the second material may comprise different soft polymers. Compression of the flange 8 during steps 108 and 110 prevents the second material 55 from overshooting the first material 50 when the second material 55 is injected into the second mould 91. This allows the first and second portions 2, 4 to retain the different properties that result from their different constituent materials 50, 55.

The first material 50 and/or the second material 55 preferably comprise thermoplastic polyurethane (TPU). TPU is advantageous because it has good chemical resistance to oils, fats and alcohols. This is particularly important for a stimulation pad, which will be exposed to fats and oils when placed into contact with the skin, and which will be cleaned with disinfectants (typically isopropanol) after use. Thus, the use of TPU results in a durable stimulation pad. A further advantage of TPU is that it is relatively soft, thereby allowing the stimulation pad 1 to be sufficiently flexible to conform to the contours of the body when in use. TPU is relatively elastic, which allows the hardness of the first portion 2 to increase when it is compressed during steps 108 and 110, and which allows the first portion 2 to revert to its original softness when the compression is released. Furthermore, TPU is a good electrical insulator. The particular composition of the TPU is not of critical importance, and the teachings disclosed herein are applicable to a wide variety of commercially-available TPU compositions.

Alternatively, the first material 50 and/or the second material 55 may comprise Styrene Ethylene Butadiene Styrene (SEBS). An advantage of SEBS is that it is available in very soft grades (down to 10 Shore A) and is easy to process. However, SEBS has limited chemical resistance to oils, fats and alcohols.

The sheath of the cable 12 preferably comprises the second material 55. This allows the sheath of the cable 12 to bond with the second material 55 when it is injected in step 110, which improves the watertightness of the stimulation pad 1 and also reduces the risk of the cable being detached from the stimulation pad 1. Preferably, the sheath of the cable 12 and the second material 55 both comprise TPU.

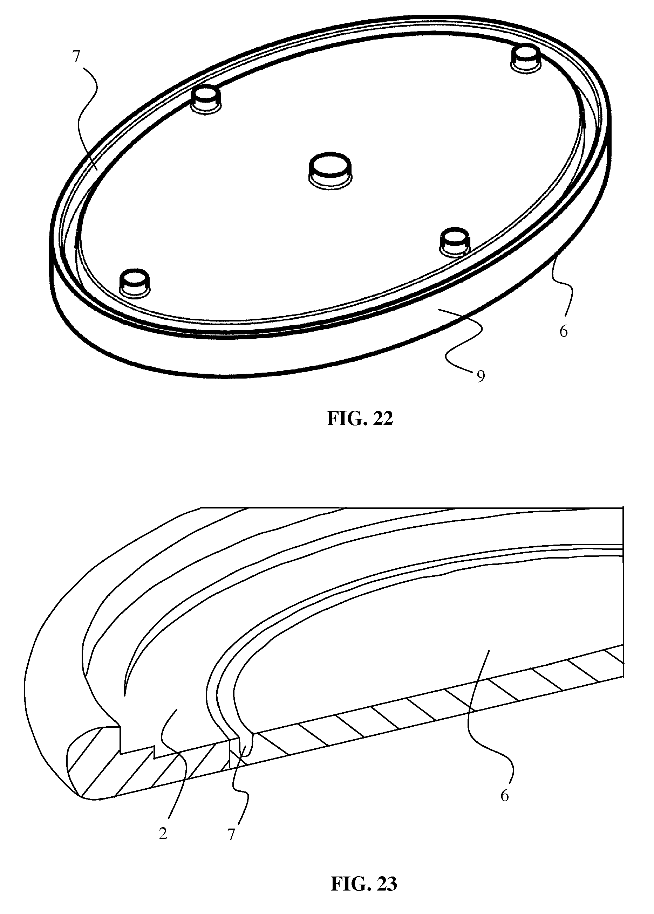

The conducting members 6a, 6b will now be described with reference to Figures 22 and 23. Figure 22 is an isometric view of a conducting member 6, whilst Figure 23 is a partial cross-sectional view of a conducting member.

Each conducting member 6 has an elliptical shape. The reason for the elliptical shape is to reduce distortion of the conducting members 6 during step 104. By way of explanation, there is a tendency for the shape of the conducting members 6 to be distorted by the compressive force that is experienced when the first material 50 is injected into the first mould 61. The inventors have discovered that a conducting member 6 is less prone to distorting when it has an elliptical shape. Furthermore, the elliptical shape also helps to promote laminar flow when the first material 50 is injected, as discussed above with reference to region 84 of Figure 12. In contrast, the inventors have found that a circular or square conducting member distorts significantly and does not bond reliably to the first material 50. Thus, the optimal shape for the conducting members 6a, 6b is an ellipse.

The conducting members 6a, 6b comprise an electrically conducting polymer. In a preferred example, the conducting members 6 comprise TPU and an electrically conducting additive. The advantage of forming the conducting members 6 from TPU is that, when the first material 50 also comprises TPU, good bonding between the conducting members 6 and the first material 50 can be achieved. The electrically conducting additive preferably comprises carbon black. The advantages of carbon black are that it is cheap, easily dispersed in the polymer matrix and does not cause skin irritation when the conducting members are placed on a human or animal body to deliver stimulation. It is possible to achieve a volume resistivity of less than 5 $\Omega$.m by adding carbon black to TPU.

As less-preferred alternatives, the electrically conducting additive could comprise carbon nanotubes or stainless steel fibres. Carbon nanotubes have a higher conductivity than carbon black and, therefore, a conducting member 6 with a particular conductivity can be produced using a smaller amount of carbon nanotubes than if carbon black were to be used, which results in a softer conducting member. However, it is difficult to disperse carbon nanotubes uniformly throughout the polymer. Stainless steel fibres have an even higher conductivity than carbon nanotubes, thereby allowing relatively soft and highly conductive conducting members to be produced. However, the steel fibres have a tendency to break when injection moulded to form the conducting member and it is difficult to disperse the steel fibres uniformly throughout the polymer.

The presence of an electrically conductive additive usually causes the hardness of a polymer to increase, which results in the conducting members 6a, 6b being harder than the first material 50. For example, when the electrically conducting additive comprises carbon black, the conducting members 6a, 6b have a hardness of 85 Shore A, whereas the hardness of the first material 50 may be 70 Shore A or even lower. Thus, the conducting members 6a, 6b are less flexible than the first portion 2, which results in a risk of the conducting members 6a, 6b detaching from the first material 50. This risk is mitigated by the groove 7 in the conducting members 6a, 6b. The groove 7 increases the flexibility of the conducting members 6a, 6b in the vicinity of their interface with the first material 50. This improves the durability of the stimulation pad 1. The groove 7 preferably has a U-shaped cross-section, as shown in Figure 23 and Detail G of Figure 24.

The conducting members 6a, 6b comprise a surface 9, to which the first material 50 is bonded. The groove 7 is formed adjacent the surface 9. The groove 7 is preferably substantially parallel to the surface 9 for substantially the whole of the surface 9. This ensures that the conducting members 6a, 6b can flex at every point at which they are bonded to the first material 50. The groove 7 is formed on an internal surface of the conducting member 6. In other words, the groove 7 is formed on the side of the conducting member 6 that faces the inside of the stimulation pad 1, i.e. the side opposite to the surface 5 of the stimulation pad 1 that makes contact with the skin when the stimulation pad 1 is in use. This allows the surfaces of the conducting members 6a, 6b that make contact with the skin to be smooth, thereby maximising the surface area that is available to conduct electricity to the human or animal body and facilitating cleaning of the stimulation pad 1.

The first protective housing 18 and second protective housing 20 each comprise a hard polymer. For example, the first and second protective housings 18, 20 preferably comprise TPU with a hardness measurable on the Shore D durometer scale. More preferably, the first and second protective housings 18, 20 comprise TPU with a hardness of approximately 75 Shore D. Advantageously, the first and second protective housings 18, 20 are able to bond to the first portion 2 and second portion 4 when all are formed from TPU. Optionally, the first protective housing 18 and/or second protective housing 20 can be potted (e.g. by filling with epoxy) before the circuit assembly is positioned on the first portion 2 of the stimulation pad 1, so as to increase the strength of the housings 18, 20. Optionally, the first and second protective housings 18, 20 can be reinforced with glass fibres if additional mechanical strength is required to withstand the pressures experienced during injection of the second material 55 in step 110. Alternatively, the first and second protective housings 18, 20 could comprise polycarbonate if yet more mechanical strength is needed.

The surfaces of the conducting members 6a, 6b can be treated prior to step 102, so as to improve their bonding to the first material 50 in step 104. Similarly, the surfaces of the first portion 2 and/or the cable 12 can be treated prior to step 106, so as to improve their bonding to the second material 55 in step 110. Plasma or corona treatment may be used to treat the surfaces. Alternatively, the surfaces can be cleaned with methyl ethyl ketone (MEK) or isopropanol.

The first mould 61 and the second mould 91 preferably have a rough surface finish. This reduces the risk of the first portion 2 or the second portion 4 sticking to the moulds 61, 91 during ejection. The resulting rough texture of the first surface 3 and the second surface 5 also advantageously helps to prevent the stimulation pad 1 from slipping whilst in use. A suitable surface finish for the first and second moulds 61, 91 is VDI-33 (MT-11530 MoldTech/Standex).

Figure 25 shows a stimulation system 200. The stimulation system 200 comprises a console 210 and a stimulation pad 1. The stimulation pad 1 is electrically connected (and, preferably, detachably connected) to the console 210 by a cable 12. In use, the stimulation pad is placed upon a human or animal body. The console 210 is operable to apply electrical stimulation to the body via the conducting members 6a, 6b. In addition to applying electrical stimulation to the body, the stimulation system 200 may also be able to apply heat to the body. That is, the stimulation system 200 can be a thermostimulation system. Such a thermostimulation system is preferably operable to apply heat and electrical stimulation to the body simultaneously or independently of each other.

An example of a circuit 16 will now be described with reference to Figures 26 to 28. Figure 26 is a top plan view of the circuit 16 shown in Figure 3. Figure 27 is a bottom plan view of the circuit 16. Figure 28 is a schematic diagram of the first connector 24 shown in Figure 3. The circuit 16 comprises a substrate 500. The substrate 500 has a first surface 53 (shown in Figure 27) and a second surface 52 (shown in Figure 26), wherein the first surface 53 has an opposite orientation to the second surface 52. The circuit 16 further comprises a heating element 502 and one or more electrodes 514. The circuit 16 can further comprise electronic components including a temperature sensor 510, a visual indicator 14 and a first connector 24.

Electrical conductors 511, 512 are patterned on each surface 52, 53 of the substrate 500 to form electrical connections between the components of the circuit 16. As used herein, the term "patterned" is preferably understood to describe the result of a process whereby an electrically conducting region having a predefined shape is formed upon a surface of the substrate 500. The conductors are illustrated by the grey shaded areas in Figures 26 and 27. The conductors on the first surface 53 are denoted by reference numeral 511 in Figure 27, whilst the conductors on the second surface 52 are denoted by reference numeral 512 in Figure 26. One or more electrodes 514 are also patterned on the first surface 53 of the substrate 500. The electrodes 514 are also illustrated by grey shaded areas in Figure 27, since the electrodes 514 are preferably formed from the same electrically conducting material as the conductors 511. Insulating regions that do not comprise a conductor are illustrated in Figures 26 and 27 by the unshaded areas denoted by reference numeral 513.

The electronic components 502, 505, 507, 510, conductors 511, 512 and electrodes 514 are provided on both surfaces 52, 53 of the substrate 500. The electrodes 514 are formed on the first surface 53, whilst the heating element 502 is formed on the second surface 52. In use, the heating element 502 faces away from the skin of the user and the electrodes 514 face towards the skin. The temperature sensor 510, visual indicator 505 and first connector 24 are also preferably provided on the second surface 52. Since electronic components 502, 505, 507, 510, conductors 511, 512 and electrodes 514 are provided on both surfaces of the substrate 500, the substrate 500 should have electrically insulating properties in order to prevent unwanted electrical conduction between components and conductors on different surfaces.

The circuit 16 comprises a first connector 24 to allow the circuit to be electrically connected to the cable 12 and thereby connected to the console 210 (shown in Figure 25). The first connector 24 is preferably provided on the second surface 52. The first connector 24 is preferably a surface-mount connector. The first connector 24 comprises connection pins, which can be connected to a corresponding connector on the cable 12. In an example, the first connector 24 comprises six connection pins, as illustrated in Figure 28. The pins labelled 'Heat+' and 'Heat-' are connected to the heating element 502. The pins labelled 'Temp+' and 'Temp-' are connected to the temperature sensor 510. The pins labelled 'EM1' and 'EM2' are connected to the electrodes 514.

The heating element 502 preferably comprises a plurality of resistors 503 and one or more conductors 512. The resistors 503 are distributed across the second surface 52 of the substrate 500. For the sake of clarity, only three resistors 503 are labelled in Figure 26; however, it can be seen that the circuit comprises many more resistors, each of which is illustrated as a small black rectangle in Figure 26. The resistors 503 are electrically connected to each other by the conductors 512. In the example illustrated in Figure 26, conductor 512a is connected to the 'Heat-' pin of the first connector 24 such that, in use, the conductor 512a operates as a negative voltage supply rail. Similarly, conductor 512b is connected to the 'Heat+' pin of the first connector 24 such that, in use, the conductor 512b operates as a positive voltage supply rail.

When a voltage is applied across the resistors 503, power is dissipated as heat. The positive and negative supply voltages are supplied to the resistors 503 by the pins labelled 'Heat+' and 'Heat-' respectively in the first connector 24. The resistors 503 are soldered to the conductors 512, and are thereby electrically connected to the first connector 24. The power dissipated by each resistor 503 is defined as:

$$P = I^2 R \qquad\qquad (1)$$

where P is the power dissipated (measured in watts), I is the current through the resistor (measured in amperes), and R is the resistance of the resistor (measured in ohms).

In an example, thirty resistors 503 are distributed over the area of the second surface 52. The resistance values of the resistors 503 preferably range from 3.3 kilohms to 6.8 kilohms in order to avoid localised areas generating more heat than surrounding regions. The resistors 503 are preferably connected in parallel, but it will be appreciated that they could also be connected in series or in a combination of series and parallel connections. In an example, a direct current input voltage of twenty-four volts is applied across the resistors 503. The invention is not limited to any particular input voltage or resistance values.

The temperature sensor 510 is mounted on the second surface 52 of the substrate 500, using surface-mount technology. The temperature sensor 510 is preferably mounted at the point equidistant between the electrodes 514a, 514b. This is to give an indication of the temperature near the region where electrical stimulation is applied, although the temperature sensor 510 could be placed at any other suitable point on the second surface 52. The positive and negative supply voltages for the temperature sensor 510 are supplied by the pins labelled 'Temp+' and 'Temp-' respectively in the first connector 24. The temperature sensor 510 is coupled to the first connector 24 by the conductors 511 patterned on the first surface 53 of the substrate 500. Vias through the substrate 500 connect the conductors 511 on the first surface 53 to the temperature sensor 510 and first connector 24 that are mounted on the second surface 52. The temperature sensor 510 can be a resistance thermometer or a thermocouple. The temperature sensor is preferably a platinum resistance thermometer (PRT), and is more preferably a Pt1000 element. A Pt1000 element is preferable due to its high accuracy.

Optionally, the resistors 503 and the temperature sensor 510 can be coated with a thin layer of TPU, prior to the circuit assembly (comprising circuit 16, first connector 24, second connector 22 and cable 12) being positioned on the first portion 2 of the stimulation pad following step 104. The thin layer of TPU may be sprayed onto the resistors 503 and 510. This provides a simple way to protect these components from being damaged by the heat and pressure experienced when the second material 55 is injected in step 110.

An electrical stimulation current is delivered from the console 20 to the electrodes 514a, 514b by the pins of the first connector 24 labelled 'EM1' and 'EM2' respectively. The electrodes 514 are coupled to the first connector 24 by the conductors 511 patterned on the first surface 53. Vias through the substrate 500 connect the conductors 511 on the first surface 53 to the first connector 24 that is mounted on the second surface 52.

Other electronic components could be mounted on the substrate 500 and, preferably, mounted on the second surface 52 of the substrate. For example, logic components such as a programmable logic device, microprocessor or microcontroller could be mounted on the substrate 500. Such logic components could be used to control the heat and/or electrical stimulation that is applied to a user. As another example, one or more sensors could be mounted on the substrate 500, in addition to the temperature sensor 510. A visual indicator 14 can be mounted on the second surface 52 of the substrate 500. The visual indicator 14 is preferably a light emitting diode.

In use, the heating element 502 faces away from the skin of the user and the electrodes 514 face towards the skin. Thus, heat generated in the heating element 502 on the second surface 52 is conducted through the substrate 500 to the first surface 53, and is subsequently conducted to the body of a user through the casing body 100 of the stimulation pad 1.

Preferably the substrate 500 is flexible and so conforms to the contours of the body when the stimulation pad 1 is placed on the body. Preferably the substrate 500 comprises plastics material and preferably the plastics material is chosen to allow the substrate 500 to be flexible. Examples of suitable materials include polyimide and polyether ether ketone (PEEK). Those skilled in the art will appreciate that the substrate 500 could comprise any other suitable material. Thus, the substrate 500, electronic components 502, 505, 507, 510, the conductors 511, 512 and the electrodes 514 collectively form a flexible circuit. Flexible circuit technology is defined by industry standards, such as IPC standards IPC-T-50, IPC-2223A and IPC-4202. Despite its flexibility, the substrate 500 is preferably substantially planar in the absence of an applied force.

As mentioned above, the moulding method described herein is used to mould stimulation pads. It is envisaged that the moulding method described herein will be particularly useful due to the method's ability to reduce distortion of the resulting article, reduce flash and ensure good bonding between the materials. By allowing one soft material to be moulded against another soft material, the obtained simulation pad is advantageously soft and flexible.

It will be understood that the invention has been described above purely by way of example, and that modifications of detail can be made within the scope of the invention as defined by the appended claims.

## Claims

1. A method (100) of moulding a pad (1) for applying stimulation to a human or animal body, comprising:

    forming a first portion of the pad by injecting (104) a first material comprising a thermoplastic material into a first mould, the first mould being shaped so as to define a flange on the first portion of the pad;
    placing (106) the first portion of the pad into a second mould, the second mould having a first plate and a second plate, wherein the second plate has a surface that is substantially parallel to a surface of the flange at all points at which the second plate abuts the flange when the second mould is closed;
    compressing (108) the flange between the first and second plates of the second mould; and
    forming a second portion of the pad by injecting (110) a second material comprising a thermoplastic material into the second mould whilst the flange is compressed between the first and second plates of the second mould.

2. A method (100) in accordance with claim 1, wherein compressing the flange causes the flange to deform, thereby creating a seal between the flange and the second mould.

3. A method (100) in accordance with claim 1 or claim 2, wherein the hardness of the first material and the hardness of the second material are each measurable on the Shore A durometer scale.

4. A method (100) in accordance with claim 3, wherein both the first material and the second material have a Shore A hardness less than or equal to 85.

5. A method (100) in accordance with claim 4, wherein at least one of the first material and the second material has a Shore A hardness of 45 to 70.

6. A method (100) in accordance with any of the preceding claims, wherein the first and second materials each comprise thermoplastic polyurethane.

7. A method (100) in accordance with any of the preceding claims, wherein the method further comprises placing an electrical circuit onto the first portion of the pad prior to forming the second portion of the pad.

8. A method (100) in accordance with claim 7, wherein the method further comprises covering at least a portion of the electrical circuit with a protective housing prior to forming the second portion of the pad.

9. A method (100) in accordance with claim 8, wherein the second material is injected into the second mould from an injection point that is oriented to direct the second material towards the protective housing.

10. A method (100) in accordance with claim 8 or claim 9, wherein the circuit is connected to a cable, wherein one end of the cable is covered by the protective housing and wherein the other end of the cable extends outside the protective housing.

11. A method (100) in accordance with claim 10, wherein the cable comprises a sheath, and wherein the protective housing is adapted to grip the sheath.

12. A method (100) in accordance with claim 10 or claim 11, wherein the protective housing is adapted to form a seal with the sheath.

13. A method (100) in accordance with any of claims 8 to 12, wherein the circuit comprises a substrate, and wherein the protective housing is adapted to form a seal with the substrate.

14. A method (100) in accordance with any of claims 7 to 13, wherein the circuit is connected to a cable, the cable comprising a sheath, and wherein the second portion of the pad is bonded to the sheath.

15. A method (100) in accordance with any of the preceding claims, wherein forming a first portion of the pad comprises:

placing an electrically conducting member into the first mould; and
injecting the first material into the first mould, such that the first material bonds to the electrically conducting member.

16. A method (100) in accordance with claim 15, wherein the electrically conducting member has an elliptical shape.

17. A method (100) in accordance with claim 15 or claim 16, wherein the electrically conducting member comprises a surface to which the first material is bonded, and a groove disposed substantially parallel to the surface.

18. A method (100) in accordance with claim 17, wherein the first mould comprises a ridge, and wherein the groove is adapted to engage with the ridge.

19. A method (100) in accordance with claim 17 or claim 18 as dependent on any of claims 7 to 14, wherein the electrical circuit comprises a substrate having a slot extending therethrough, wherein the groove is aligned with said slot when the electrical circuit is placed onto the first portion of the pad.

20. A method (100) in accordance with any of the preceding claims, wherein the first mould comprises an insert, the insert being separated from the first mould by a gap, wherein the gap allows air to exit the first mould when the first material is injected into the first mould.

21. A stimulation pad (1) produced by a method in accordance with any of the preceding claims.

**Patentansprüche**

1. Verfahren (100) zum Formen eines Pads (1) zum Ausüben von Stimulation auf einen menschlichen oder tierischen Körper, das Folgendes umfasst:

   Bilden eines ersten Abschnitts des Pads durch Einspritzen (104) eines ein thermoplastisches Material umfassenden ersten Materials in ein erstes Formwerkzeug, wobei das erste Formwerkzeug derart geformt ist, dass es einen Flansch an dem ersten Abschnitt des Pads definiert;
   Platzieren (106) des ersten Abschnitts des Pads in ein zweites Formwerkzeug, wobei das zweite Formwerkzeug eine erste Platte und eine zweite Platte aufweist, wobei die zweite Platte eine Oberfläche aufweist, die an allen Stellen, an denen die zweite Platte an dem Flansch anliegt, wenn das zweite Formwerkzeug geschlossen ist, im Wesentlichen parallel zu einer Oberfläche des Flanschs ist;
   Zusammendrücken (108) des Flanschs zwischen der ersten und der zweiten Platte des zweiten Formwerkzeugs; und
   Bilden eines zweiten Abschnitts des Pads durch Einspritzen (110) eines ein thermoplastisches Material umfassenden zweiten Materials in das zweite Formwerkzeug, während der Flansch zwischen der ersten und der zweiten Platte des zweiten Formwerkzeugs zusammengedrückt wird.

2. Verfahren (100) nach Anspruch 1, wobei das Zusammendrücken des Flanschs bewirkt, dass sich der Flansch verformt, wodurch eine Dichtung zwischen dem Flansch und dem zweiten Formwerkzeug erzeugt wird.

3. Verfahren (100) nach Anspruch 1 oder Anspruch 2, wobei die Härte des ersten Materials und die Härte des zweiten Materials jeweils auf der Shore A-Durometerskala messbar sind.

4. Verfahren (100) nach Anspruch 3, wobei sowohl das erste Material als auch das zweite Material eine Shore A-Härte von weniger als oder gleich 85 aufweisen.

5. Verfahren (100) nach Anspruch 4, wobei das erste Material und/oder das zweite Material eine Shore A-Härte von 45 bis 70 aufweist.

6. Verfahren (100) nach einem der vorangehenden Ansprüche, wobei das erste und das zweite Material jeweils thermoplastisches Polyurethan aufweisen.

7. Verfahren (100) nach einem der vorangehenden Ansprüche, wobei das Verfahren weiter das Platzieren einer elektrischen Schaltung auf den ersten Abschnitt des Pads vor dem Bilden des zweiten Abschnitts des Pads umfasst.

8. Verfahren (100) nach Anspruch 7, wobei das Verfahren weiter das Abdecken von mindestens einem Abschnitt der elektrischen Schaltung mit einem Schutzgehäuse vor dem Formen des zweiten Abschnitts des Pads umfasst.

9. Verfahren (100) nach Anspruch 8, wobei das zweite Material von einer Einspritzstelle in das zweite Formwerkzeug eingespritzt wird, die dazu orientiert ist, das zweite Material in Richtung auf das Schutzgehäuse zu lenken.

10. Verfahren (100) nach Anspruch 8 oder Anspruch 9, wobei die Schaltung mit einem Kabel verbunden ist, wobei ein Ende des Kabels von dem Schutzgehäuse abgedeckt ist und wobei sich das andere Ende des Kabels außerhalb des Schutzgehäuses erstreckt.

11. Verfahren (100) nach Anspruch 10, wobei das Kabel einen Mantel umfasst und wobei das Schutzgehäuse dazu angepasst ist, den Mantel zu greifen.

12. Verfahren (100) nach Anspruch 10 oder Anspruch 11, wobei das Schutzgehäuse dazu angepasst ist, mit dem Mantel eine Dichtung zu bilden.

13. Verfahren (100) nach einem der Ansprüche 8 bis 12, wobei die Schaltung ein Substrat umfasst und wobei das Schutzgehäuse dazu angepasst ist, mit dem Substrat eine Dichtung zu bilden.

14. Verfahren (100) nach einem der Ansprüche 7 bis 13, wobei die Schaltung mit einem Kabel verbunden ist, wobei das Kabel einen Mantel umfasst und wobei der zweite Abschnitt des Pads an dem Mantel haftet.

**15.** Verfahren (100) nach einem der vorangehenden Ansprüche, wobei das Bilden eines ersten Abschnitts des Pads Folgendes umfasst:

Platzieren eines elektrisch leitenden Elements in das erste Formwerkzeug; und
Einspritzen des ersten Materials in das erste Formwerkzeug, sodass das erste Material an dem elektrisch leitenden Element haftet.

**16.** Verfahren (100) nach Anspruch 15, wobei das elektrisch leitende Element eine elliptische Form aufweist.

**17.** Verfahren (100) nach Anspruch 15 oder Anspruch 16, wobei das elektrisch leitende Element eine Oberfläche, an der das erste Material haftet, und eine zu der Oberfläche im Wesentlichen parallel angeordnete Nut umfasst.

**18.** Verfahren (100) nach Anspruch 17, wobei das erste Formwerkzeug einen Wulst umfasst und wobei die Nut dazu angepasst, mit dem Wulst in Eingriff zu treten.

**19.** Verfahren (100) nach Anspruch 17 oder Anspruch 18, wenn abhängig von einem der Ansprüche 7 bis 14, wobei die elektrische Schaltung ein Substrat mit einem sich dadurch hindurch erstreckenden Schlitz umfasst, wobei die Nut mit dem Schlitz ausgerichtet ist, wenn die elektrische Schaltung auf den ersten Abschnitt des Pads platziert wird.

**20.** Verfahren (100) nach einem der vorangehenden Ansprüche, wobei das erste Formwerkzeug einen Einsatz umfasst, wobei der Einsatz durch einen Spalt von dem ersten Formwerkezug getrennt ist, wobei der Spalt Luft aus dem ersten Formwerkzeug austreten lässt, wenn das erste Material in das erste Formwerkzeug eingespritzt wird.

**21.** Stimulationspad (1), das durch ein Verfahren gemäß einem der vorangehenden Ansprüche hergestellt wird.

**Revendications**

**1.** Procédé (100) de moulage d'une pastille (1) pour appliquer une stimulation sur le corps d'une personne ou d'un animal, comprenant :

la formation d'une première partie de la pastille en injectant (104) une première matière comprenant une matière thermoplastique dans un premier moule, le premier moule étant conformé de manière à définir une bride sur la première partie de la pastille ;
le placement (106) de la première partie de la pastille dans un second moule, le second moule ayant une première plaque et une seconde plaque, la seconde plaque ayant une surface sensiblement parallèle à une surface de la bride à tous les points auxquels la seconde plaque jouxte la bride quand le second moule est fermé ;
la compression (108) de la bride entre les première et seconde plaques du second moule ; et
la formation d'une seconde partie de la pastille en injectant (110) une seconde matière comprenant une matière thermoplastique dans le second moule pendant que la bride est compressée entre les première et seconde plaques du second moule.

**2.** Procédé (100) selon la revendication 1, dans lequel la compression de la bride entraîne une déformation de la bride, créant ainsi un joint hermétique entre la bride et le second moule.

**3.** Procédé (100) selon la revendication 1 ou la revendication 2, dans lequel la dureté de la première matière et la dureté de la seconde matière sont chacune mesurables sur l'échelle d'un duromètre Shore A.

**4.** Procédé (100) selon la revendication 3, dans lequel la première matière et la seconde matière ont toutes les deux une dureté Shore A inférieure ou égale à 85.

**5.** Procédé (100) selon la revendication 4, dans lequel au moins l'une de la première matière et de la seconde matière a une dureté Shore A de 45 à 70.

**6.** Procédé (100) selon l'une quelconque des revendications précédentes, dans lequel les première et seconde matières comprennent chacune un polyuréthane thermoplastique.

**7.** Procédé (100) selon l'une quelconque des revendications précédentes, le procédé comprenant en outre le placement d'un circuit électrique sur la première partie de la pastille avant de former la seconde partie de la pastille.

8. Procédé (100) selon la revendication 7, le procédé comprenant en outre le recouvrement d'au moins une partie du circuit électrique avec un boîtier de protection avant de former la seconde partie de la pastille.

9. Procédé (100) selon la revendication 8, dans lequel la seconde matière est injectée dans le second moule à partir d'un point d'injection qui est orienté pour diriger la seconde matière vers le boîtier de protection.

10. Procédé (100) selon la revendication 8 ou la revendication 9, dans lequel le circuit est connecté à un câble, dans lequel une extrémité du câble est recouverte par le boîtier de protection et dans lequel l'autre extrémité du câble s'étend en dehors du boîtier de protection.

11. Procédé (100) selon la revendication 10, dans lequel le câble comprend une gaine, et dans lequel le boîtier de protection est adapté pour saisir la gaine.

12. Procédé (100) selon la revendication 10 ou la revendication 11, dans lequel le boîtier de protection est adapté pour former un joint étanche avec la gaine.

13. Procédé (100) selon l'une quelconque des revendications 8 à 12, dans lequel le circuit comprend un substrat, et dans lequel le boîtier de protection est adapté pour former un joint étanche avec le substrat.

14. Procédé (100) selon l'une quelconque des revendications 7 à 13, dans lequel le circuit est connecté à un câble, le câble comprend une gaine, et dans lequel la seconde partie de la pastille est collée à la gaine.

15. Procédé (100) selon l'une quelconque des revendications précédentes, dans lequel la formation d'une première partie de la pastille comprend :

le placement d'un élément électriquement conducteur dans le premier moule ; et
l'injection de la première matière dans le premier moule, de telle sorte que la première matière se colle à l'élément électriquement conducteur.

16. Procédé (100) selon la revendication 15, dans lequel l'élément électriquement conducteur a une forme elliptique.

17. Procédé (100) selon la revendication 15 ou la revendication 16, dans lequel l'élément électriquement conducteur comprend une surface à laquelle la première matière est collée, et une rainure disposée sensiblement parallèlement à la surface.

18. Procédé (100) selon la revendication 17, dans lequel le premier moule comprend une nervure, et dans lequel la rainure est adaptée pour se mettre en prise avec la nervure.

19. Procédé (100) selon la revendication 17 ou la revendication 18 lorsque dépendant de l'une quelconque des revendications 7 à 14, dans lequel le circuit électrique comprend un substrat traversé par une fente, dans lequel la rainure est alignée avec ladite fente quand le circuit électrique est placé sur la première partie de la pastille.

20. Procédé (100) selon l'une quelconque des revendications précédentes, dans lequel le premier moule comprend un insert, l'insert étant séparé du premier moule par un espace, dans lequel l'espace permet à l'air de sortir du premier moule quand la première matière est injectée dans le premier moule.

21. Pastille de stimulation (1) produite par un procédé selon l'une quelconque des revendications précédentes.

**FIG. 1**

**FIG. 2**

**FIG. 3**

**FIG. 4**

100

Insert components into first mould — 102

Inject first material into first mould, to form first portion of article — 104

Place first portion of article into second mould — 106

Compress flange on first portion of article with second mould — 108

Inject second material into second mould, to form second portion of article — 110

**FIG. 5**

**FIG. 6**

**FIG. 7**

**FIG. 8**

EP 2 830 849 B1

**FIG. 9**

**FIG. 10**

FIG. 11

FIG. 12

**FIG. 13**

**FIG. 14**

**FIG. 15**

**FIG. 16**

**FIG. 17**

**FIG. 18**

**FIG. 20**

**FIG. 19**

**FIG. 21**

**FIG. 22**

**FIG. 23**

FIG. 24

Detail B

Detail C

Detail D

Detail E

Detail F

Detail G

Detail H

**FIG. 25**

Heat+

EM1

Temp+

Temp-

EM2

Heat-

24

**FIG. 28**

FIG. 26

FIG. 27

**EP 2 830 849 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2011150092 A
- US 7739791 B
- US 6319448 B
- US 2005116384 A
- US 2011125204 A
- DE 102005020200